Europäisches Patentamt

European Patent Office

Office européen des brevets

(19)

(11) Publication number: **0 038 060**
**B1**

(12) **EUROPEAN PATENT SPECIFICATION**

(45) Date of publication of patent specification: **11.01.84**

(21) Application number: **81102809.1**

(22) Date of filing: **13.04.81**

(51) Int. Cl.³: **A 61 K 31/615,**
**A 61 K 31/60,**
**A 61 K 31/375,**
**A 61 K 31/195** //(A61K31/60,
31/375, 31/195, 31/12)

(54) A pharmaceutical composition for treating hypertension containing L-alpha-methyl-3,4–dihydroxyphenylalanine and salicylamide.

(30) Priority: **14.04.80 US 139913**

(43) Date of publication of application:
**21.10.81 Bulletin 81/42**

(45) Publication of the grant of the patent:
**11.01.84 Bulletin 84/2**

(84) Designated Contracting States:
**AT BE CH DE FR GB IT LI LU NL SE**

(56) References cited:
**EP - A - 0 011 868**
**FR - A - 2 159 413**
**US - A - 3 526 698**

(73) Proprietor: **MERCK & CO. INC.**
**126, East Lincoln Avenue P.O. Box 2000**
**Rahway New Jersey 07065 (US)**

(72) Inventor: **Vickers, Stanley**
**Box 243 R.D. 2**
**Perkasie Pennsylvania 18944 (US)**

(74) Representative: **Abitz, Walter, Dr.-Ing. et al,**
**Abitz, Morf, Gritschneder P.O. Box 86 01 09**
**D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

A pharmaceutical composition for treating hypertension containing L-α-methyl-3,4-dihydroxyphenylalanine and salicylamide

The present invention concerns a pharmaceutical composition for treating hypertension. The components are L-α-methyl-3,4-dihydroxyphenylalanine and salicylamide.

L-α-methyl-3,4-dihydroxyphenylalanine (α-methyldopa) is a commercial antihypertensive agent [see Merck Index 9th Ed., Item 5925 (1976)]. Salicylamide is a commercial analgesic [Merck Index 9th Ed., Item 8080 (1976)].

The combination of methyldopa with salicylamide has been found to have enhanced antihypertensive properties.

FR—A—2 159 415 describes a composition comprising a salt of α-methyldopa, e.g. a salt with ascorbic acid. US—A—3 526 698 suggests to incorporate an antioxidant, e.g. ascorbic acid, into a composition comprising α-methyldopa. Statements which could show that the compositions of the present invention might have an antihypertensive effect superior to that of α-methyldopa are not contained in either of the citations.

The non-prepublished EP—A—0 011 868 describes pharmaceutical compositions for the oral treatment of hypertension comprising an aqueous suspension containing methyldopa and sucrose.

The subject matter of the present invention is a pharmaceutical composition for treating hypertension containing (A) L-α-methyl-3,4-dihydroxyphenylalanine (α-methyldopa) and (B) salicylamide, wherein the weight ratio of (A):(B) is at least about 3:1.

The (A):(B) weight ratio in the composition of the present invention may be varied. A preferred weight ratio of (A):(B) is 16:1 to 3:1. A more preferred (A):(B) weight ratio is 12:1 to 3:1. A still more preferred weight ratio is 8:1 to 4:1.

The compositions of the present invention may be administered either orally or parenterally in treating hypertension in humans. Appropriate dosage forms are used. Oral administration is preferred. Suitable oral dosage forms may be solid e.g. tablets, troches, capsules and the like, or liquid e.g. solution, suspension, emulsion and the like. Where necessary, or desirable, common pharmaceutically acceptable compounding ingredients i.e. diluents, carriers, processing acids, etc. may be used in preparing the dosage forms. Conventional procedures, compounding ingredients and equipment are used to prepare the oral and/or parenteral dosage forms.

The weight ratio of the (A) and (B) components in the dosage form will be within the ranges already set out above.

In treating hypertension in humans, an anti-hypertensive effective amount of the composition containing the (A) and (B) components is administered. The daily dosage of the composition will be varied depending on various factors e.g. the severity of the hypertension (high blood pressure), the weight of the patient etc. Generally, daily dosages may range up to about 3000 mg, preferably from 250 mg to 2000 mg, and more preferably from 500 mg to 1500 mg.

The enhanced effectiveness of the present composition as an antihypertensive agent was demonstrated in an *in vivo* test using spontaneously hypertensive (SH) rats. The data for this *in vivo* test is tabulated below:

TABLE 1

ANTIHYPERTENSIVE ACTIVITY IN SH RAT
ORAL ADMINISTRATION

| Test No. | Composition Containing | Dose (mg/kg) | No. of Rats | Mean Arterial Pressure[1] (mm/Hg) Hours After Treatment | | | | | | | |
|---|---|---|---|---|---|---|---|---|---|---|---|
| | | | | 0 | 1/2 | 1 | 2 | 4 | 8 | 12 | 18 |
| 1 | α-Methyldopa | 80 | 10 | 164 | 155 | 164 | 148 | 141 | 152 | 158 | 160 |
| 2 | α-Methyldopa | 80 | 16 | 173 | 178 | 172 | 163 | 152 | 154 | 166 | 169 |
| 3 | { α-Methyldopa + Salicylamide | 80 + 10 | 5 | 174 | 162 | 161 | 153 | 142 | 136 | 150 | 148 |
| 4 | { α-Methyldopa + Salicylamide | 80 + 20 | 3 | 186 | 172 | 170 | 167 | 154 | 170 | 168 | 176 |
| 5 | { α-Methyldopa + Salicylamide | 80 + 40 | 2 | 164 | 144 | 158 | 156 | 158 | 160 | 158 | 160 |

[1] Average

2

**0 038 060**

The data shows that the combination of $\alpha$-methyldopa and salicylamide, weight ratio 8:1 and 4:1, is more effective than $\alpha$-methyldopa in reducing mean arterial pressure of SH rats. This indicates that the compositions of the present invention would be more effective than $\alpha$-methyldopa in treating hypertensive humans.

**Claims for the Contracting States: BE CH DE FR GB IT LI LU NL SE**

1. A pharmaceutical composition for treating hypertension containing (A) L-$\alpha$-methyl-3,4-dihydroxyphenylalanine and (B) salicylamide wherein the weight ratio of (A):(B) is at least about 3:1.

2. The pharmaceutical composition of Claim 1 wherein the weight ratio of L-$\alpha$-methyl-3,4-dihydroxyphenylalanine:salicylamide is 3:1 to 16:1.

3. The pharmaceutical composition of Claim 2 wherein said ratio is 4:1 to 8:1.

4. The pharmaceutical composition of Claim 3 wherein said ratio is 4:1.

5. The pharmaceutical composition of Claim 3 wherein said ratio is 8:1.

**Claims for the Contracting State: AT**

1. A process for preparing a pharmaceutical composition for treating hypertension comprising combining (A) L-$\alpha$-methyl-3,4-dihydroxyphenylalanine and (B) salicylamide in a weight ratio of (A):(B) of at least about 3:1.

2. The process of Claim 1 wherein the weight ratio of L-$\alpha$-methyl-3,4-dihydroxyphenylalanine:salicylamide is 3:1 to 16:1.

3. The process of Claim 2 wherein said ratio is 4:1 to 8:1.

4. The process of Claim 3 wherein said ratio is 4:1.

5. The process of Claim 3 wherein said ratio is 8:1.

**Revendications pour les Etats contractants: BE CH DE FR GB IT LI LU NL SE**

1. Composition pharmaceutique pour le traitement de l'hypertension contenant (A) de la L-$\alpha$-méthyl-3,4-dihydroxyphénylalanine et (B) du salicylamide dans laquelle le rapport pondéral de (A)/(B) est d'au moins environ 3/1.

2. La composition pharmaceutique de la revendication 1, dans laquelle le rapport pondéral de la L-$\alpha$-méthyl-3,4-dihydroxyphénylalanine au salicylamide est de 3/1 à 16/1.

3. La composition pharmaceutique de la revendication 2, dans laquelle ledit rapport est de 4/1 à 8/1.

4. La composition pharmaceutique de la revendication 3, dans laquelle ledit rapport est de 4/1.

5. La composition pharmaceutique de la revendication 3, dans laquelle ledit rapport est de 8/1.

**Revendications pour l'Etat contractant: AT**

1. Un procédé pour la préparation d'une composition pharmaceutique pour le traitement de l'hypertension comprenant la combinaison de (A) L-$\alpha$-méthyl-3,4-dihydroxyphénylalanine et (B) du salicylamide dans un rapport pondéral de (A)/(B) d'au moins environ 3/1.

2. Le procédé de la revendication 1, dans lequel le rapport pondéral de la L-$\alpha$-méthyl-3,4-dihydroxyphénylalanine au salicylamide est de 3/1 à 16/1.

3. Le procédé de la revendication 2, dans lequel ledit rapport est de 4/1 à 8/1.

4. Le procédé de la revendication 3, dans lequel ledit rapport est de 4/1.

5. Le procédé de la revendication 3, dans lequel ledit rapport est de 8/1.

**Patentansprüche für die Vertragsstaaten: BE CH DE FR GB IT LI LU NL SE**

1. Eine pharmazeutische Zusammensetzung zur Behandlung hohen Blutdrucks, enthaltend (A) L-$\alpha$-Methyl-3,4-dihydroxyphenylalanin und (B) Salicylamid, worin das Gewichtsverhältnis von (A):(B) wenigstens etwa 3:1 ist.

2. Die pharmazeutische Zusammensetzung des Anspruchs 1, worin das Gewichtsverhältnis von L-$\alpha$-Methyl-3,4-dihydroxyphenylalanin:Salicylamid 3:1 bis 16:1 ist.

3. Die pharmazeutische Zusammensetzung des Anspruchs 2, worin dieses Verhältnis 4:1 bis 8:1 ist.

4. Die pharmazeutische Zusammensetzung des Anspruchs 3, worin dieses Verhältnis 4:1 ist.

5. Die pharmazeutische Zusammensetzung des Anspruchs 3, worin dieses Verhältnis 8:1 ist.

3

**0 038 060**

**Patentansprüche für den Vertragsstaat: AT**

1. Ein Verfahren zur Herstellung einer pharmazeutischen Zusammensetzung für die Behandlung hohen Blutdrucks, umfassend die Vereinigung von (A) L-$\alpha$-Methyl-3,4-dihydroxyphenylalanin und (B) Salicylamid in einem Gewichtsverhältnis von (A):(B) von wenigstens etwa 3:1.

2. Das Verfahren des Anspruchs 1, worin das Gewichtsverhältnis von L-$\alpha$-Methyl-3,4-dihydroxy-phenylalanin:Salicylamid 3:1 bis 16:1 ist.

3. Das Verfahren des Anspruchs 2, worin dieses Verhältnis 4:1 bis 8:1 ist.

4. Das Verfahren des Anspruchs 3, worin dieses Verhältnis 4:1 ist.

5. Das Verfahren des Anspruchs 3, worin dieses Verhältnis 8:1 ist.